# EUROPEAN PATENT SPECIFICATION

(11) **EP 3 118 329 B1**
(45) Date of publication and mention of the grant of the patent: **15.05.2019**
(21) Application number: 16179021.7
(22) Date of filing: 12.07.2016
(51) Int. Cl.: C12Q 1/682, C12Q 1/6827, C12Q 1/686, C12Q 1/6886

(54) **PROBES AND A METHYLATION IN SITU HYBRIDIZATION ASSAY**
SONDEN UND EIN IN-SITU-HYBRIDISIERUNGS-ASSAY DER METHYLIERUNG
SONDES ET TEST D'HYBRIDATION PAR MÉTHYLATION IN SITU

(30) Priority: 15.07.2015 EP 15176744
(43) Date of publication of application: 18.01.2017
(73) Proprietor: Universiteit Gent, 9000 Gent (BE)
(72) Inventor: LIEVELD, Marusya, 9890 Gavere (BE); VAN CRIEKINGE, Wim, 2550 Waarloos (BE); VANDEN BROECK, Davy, 2811 Leest (BE)

(56) References cited:
- EP-A2- 1 264 899
- WO-A1-2013/007702
- WO-A2-2008/093336
- WO-A2-2009/076478
- US-A- 6 046 038
- US-A1- 2007 087 360
- US-A1- 2012 004 132
- WANG Y ET AL: "In situ bisulfite modification of membrane-immobilized DNA for multiple methylation analysis", ANALYTICAL BIOCHEMISTRY, ACADEMIC PRESS INC, NEW YORK, vol. 359, no. 2, 15 December 2006 (2006-12-15), pages 183-188, XP024942241, ISSN: 0003-2697, DOI: 10.1016/J.AB.2006.09.001 [retrieved on 2006-12-15]
- NURMI J ET AL: "A new label technology for the detection of specific polymerase chain reaction products in a closed tube", NUCLEIC ACIDS RESEARCH, INFORMATION RETRIEVAL LTD, GB, vol. 28, no. 8, 1 January 2000 (2000-01-01), pages E28)1-V1, XP002955418, ISSN: 0305-1048, DOI: 10.1093/NAR/28.8.E28
- J. H. Do ET AL: "cDNA Labeling Strategies for Microarrays Using Fluorescent Dyes", ENGINEERING IN LIFE SCIENCES, vol. 7, no. 1, 1 February 2007 (2007-02-01), pages 26-34, XP055435788, DE ISSN: 1618-0240, DOI: 10.1002/elsc.200620169
- Didenko V. Vladimir ET AL: "Selection of Fluorophore and Quencher Pairs for Fluorescent Nucleic Acid Hybridization Probes" In: "Fluorescent Energy Transfer Nucleic Acid Probes", 1 April 2006 (2006-04-01), Humana Press, New Jersey, XP055193619, ISBN: 978-1-58829-380-0 vol. 335, pages 3-16, DOI: 10.1385/1-59745-069-3:3,

## Description

### Technical field of invention

The present invention relates to the field of molecular pathology (for example cancer diagnosis, prognosis, treatment and/or therapy prediction) through the detection of RNA, mutations, copy number changes and determination of the methylation status of specific sequences of the genome of individual patients in hybridization assays (southern blot, ISH, dot blot) including *in situ* determination of the methylation status of specific sequences of the genome of individual patients in individual cells. More specifically the present invention relates to: a) target-specific probes covalently-attached to a labeled tail, b) the synthesis method of said probe, c) the usage of said probe such as an *in situ* hybridization-based method to correlate the methylation status of a promoter region of a gene in a biopsy or cytology specimen of a patient to the morphology and localization in that specimen, and d) kits comprising said target-specific probes. The latter method and products allow detection of (epi) genetic changes in specific cell types of histological or cytological (cancer) specimens or on membranes which will contribute to scientific research and which will help physicians to accurately diagnose diseases and/or start an appropriate treatment.

### Background art

The following target-specific probes or signal amplification systems for *in situ* hybridization have been described:

### 1) Padlock probe - rolling circle amplification (RCA) (Larsson et al., 2004):

This method combines PCR amplification for sufficient signal amplification and enzymatic restriction to allow probe access; and does thus not allow quantification of the target at physiological levels.

In RCA the target DNA is restriction digested at the 3'end of the target sequence and irreversibly made single stranded by strand-specific 5'-3' exonucleolysis. Padlock probes are hybridized to their target sequences and the probe ends are joined through ligation, locking the probe onto the target molecule. After ligation, the RCA is initiated by the F29 DNA polymerase by turning the target molecule into a primer through 3'-5' exonucleolysis of any 3' end protruding beyond the padlock probe hybridization site. The padlock probe then serves as the template for DNA synthesis. The RCA product is detected through hybridization of fluorescence-labeled oligonucleotides to tag sequences, specific for the padlock probe (Larsson *et al.,* 2004).

The sensitivity of this technique is only 10% since the enzymatic restriction step that exposes the sense or anti-sense strand is not absolute and is difficult to regulate; further factors contributing to the reduced sensitivity is the low PCR efficiency and DNA loss.

RCA is thus based on amplification of the target-specific probe and detection of the amplified material.

Moreover, the detection probes used in RCA are molecular inversion probes (MIP).

The probes are designed with complementary sequences to the target at its 5' and 3' ends. The internal region contains two universal PCR primer sites that are common to all MIPs as well as a probe-release site, which is usually a restriction site.

### 2) Lollipop probes for signal amplification (US20020192658)

A lollipop oligomer is a branched oligomer that comprises tail portion, a right arm portion, and a left arm portion. The two arms each end with sequences complementary to adjacent sequences in a target sequence. This allows the right and left arms to be ligated together when the oligomer is hybridized to the target sequence. The tail portion comprises a rolling circle replication primer, amplification of the signal is then performed by means of RCA. The tail portion can then be detected at the location of the target sequence.

### 3) Branched DNA amplification (bDNA) (Collins et al., 1997):

The bDNA protocol includes four probe hybridization steps followed by sufficient washing after each step. First a target-specific probe containing a small toe is added to the sample followed by a second hybridization with a pre-amplifier oligo that will bind the target-specific probe. A third hybridization with the amplifier probe that will bind the pre-amplifier is then performed. Finally, labeled probes that will hybridize with the amplifier are added. The bDNA system is based on a four-step hybridization protocol to create an amplification tree that gives sufficient signal for target probe detection. On the contrary, the probe of the present invention is an one-molecule probe that allows target-detection in an "one hybridization step" protocol.

bDNA is composed of four single-stranded oligomers that hybridize with each other to create an amplification tree. The probe of the present invention consists out of one molecule that contains a target-specific part and a signal amplification part.

Aspecific binding of the branched molecules in bDNA is prevented by including isobases in their sequences.

### 4) Tyramide signal amplification (TSA) system (Schriml et al., 1999):

TSA is an enzyme-mediated detection method that uses horseradish peroxidase (HRP) for signal amplification. In this system biotin-labeled probes are hybridized with the target following addition of streptavidin-HRP. Tyramide-fluorophore are deposited by HRP in the amplification reaction.

Signal amplification of target-specific probes is performed in two steps and is generated by an enzymatic process (deposition of labeled tyramide) whereas the present invention relates to signal amplification by means of a strongly labeled signal tail sequence linked to a target-specific part.

Probes used for TSA amplification -in contrary to the probes of the present invention- do not contain a signal amplification part.

Overall drawbacks of the above mentioned alternative methods and probes are their complex and expensive protocols, low sensitivity, low quantification possibilities, various signal amplification steps after probe hybridization and extensive washing steps, resulting in target lost and a high background staining which hamper their use for routine application. There is thus a need to design better performing probes which are capable to visualize targets in hybridization assays and which can be used for routine application.
5) The following methods that may allow to detect methylation changes have been described:
5.1 Nuovo *et al.* (Nuovo *et al.,* 1999) disclose methylation specific-PCR *in situ* hybridization (ISH). They monitored p16^{INK4a} methylation changes in Formalin-Fixed, Paraffin-Embedded (FFPE) tissue samples. In this protocol *in situ* bisulfite conversion is first performed overnight, followed by methylation-specific in situ PCR (MSP) MSP uses primers specific for detection of sequence differences between methylated versus unmethylated DNA, that result from bisulfite modification; Bisulfite modified DNA was amplified with p16^{INK4a} gene specific primers 5'-TTTTTAGAGGATTTGAGGGATAGG-'3 (sense, SEQ ID N° 1) and 5'- CTACCTAATTCCAATTCCCCTACA-'3 (anti-sense, SEQ ID N° 2). After amplification, *in situ* hybridization was performed by simultaneously adding long (>80 base pairs (bp) sized) unmethylated-specific or methylated-specific internally digoxigenin labeled probes. Here again, PCR amplification is used for signal amplification and the efficiency of the test relies on PCR specificity followed by amplicon detection by probes. This test does thus not allow detection of the target at physiological levels. Moreover, because the target is amplified and the amplicons will crowd the nuclei, co-localization of multiple targets (for example an unmethylated target and a methylated target) will be very difficult to interpreted.
5.2 Larsson *et al.* (Larsson *et al.,* 2004) describe padlock probes for single-nucleotide polymorphisms (SNPs) detection. These are oligonucleotide probes that induce circularization of the target after hybridization to the target region. Double stranded (dsDNA) is made accessible for padlock probe hybridization by enzymatic digestion. A combination of restriction enzymes and exonuclease enzymes (MSCI and EcoRV) is used. Following PCR amplification, labeled oligo probes are added and these recognize the amplified target. However, the efficiency of the probe hybridization is only 10% because enzymatic restriction that should, expose the sense or anti-sense strand is not absolute and it is difficult to regulate, further factors are PCR efficiency and DNA loss. This method thus combines PCR amplification for sufficient signal amplification and enzymatic restriction to allow probe access; and thus not allow quantification of the target at physiological levels.
5.3 Li *et al.* (Li *et al.,* 2013) disclose microscopic evaluation of the methylation status at satellite repeats. This paper demonstrates the detection of the methylation status of minor and major satellite repeats using labeled Locked Nucleic Acids (LNA) probes. Probe recognition depends on crosslinking of a bipyridine-adenine derivative at the position corresponding to the methylated cytosine in the presence of osmium, therefore, the described method does not allow detection of unmethylated sequences; and so hypomethylation cannot be observed. The described method can only be used for detection of highly abundant repeats, because these small probes cannot compete with re-hybridization of the complementary strands and will not generate enough signals for microscopic evaluation of single copy genes.

In order to ensure a sufficient sensitivity for microscopic evaluation, the above-described methods must either be PCR-based (Larsson *et al.,* 2004; Nuovo *et al.,* 1999) or they can only detect abundant targets such as satellite repeats (Li *et al.,* 2013). Specificity is achieved by target-specific amplification with methylation-specific primers, following *in situ* hybridization with >80 bp probes (Nuovo *et al.,* 1999), crosslinking by means of a bipyridine-adenine derivative at the position corresponding to the methylated cytosine in the presence of osmium (Li *et al.,* 2013) or ligation of padlock probes at SNP positions (Larsson *et al.,* 2004).

US 2007/00873360 discloses the probes 'P15 Me FAM B-OLA' and 'P15 UnMe ASO'. Both of said probes consist solely of a target-specific nucleic acid sequence of which 1 single nucleotide is labeled with FAM (Fluorescein amidite).

US 6,046,038 discloses a probe consisting of 'a single strand # 3' and an 'extension mer' which is attached to 'the strand labeled during polymerization'. The latter 'extension mer' is a template used to synthesize said 'strand labeled during polymerization'.

WO 2009/076478 discloses probes wherein the target-specific part is labeled in its complete extension and to probes which are single-labeled or dual-labeled at one of both ends of the probe. US2012/0004132 discloses the usage of a combination of 4 probes of which only one probe comprises one single lable.

### Brief description of figures

**Figure 1****:** Schematic representation of target-specific probe consisting out of a target-specific compound (thick bar) and a labeled compound (thin bar, light grey and stars). The labeled compound can be sealed by an unlabeled complementary sequence (thin bar dark grey).

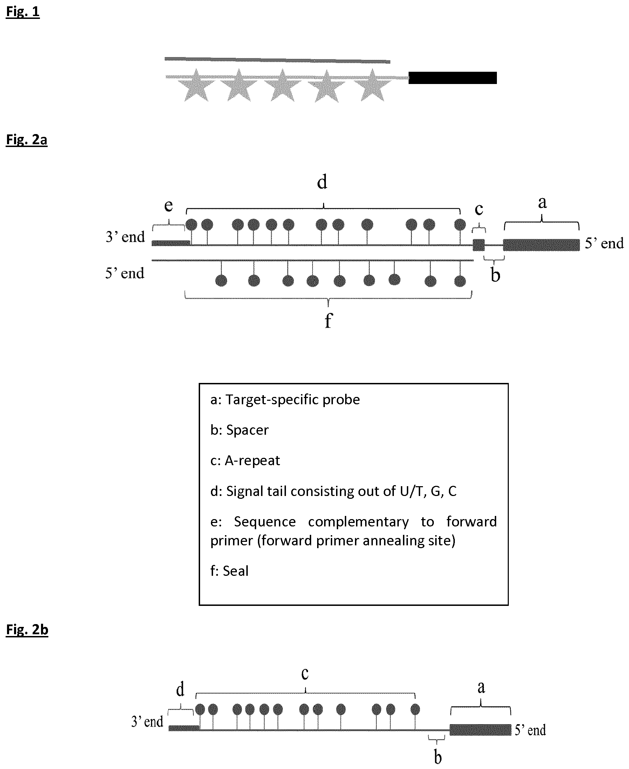

**Figure 2****: a)** A sealed, target specific probe or Uniprobe Signal Amplification System (UPSAS) of the invention consists out of 6 major parts: a) a target-specific probe sequence with a similar sequence as the reverse primer (RP), b) a spacer of at least one nucleotide, c) an A-stretch of at least one nucleotide (this is included to stop probe sealing when probe sealing is performed with three nucleotides), d) a labeled part that consists out of UGC or TGC nucleotides (this is the signal tail), e) a sequence that is complementary to the forward primer used in probe synthesis, and f) a 'seal' consisting out of a complementary sequence of the RP and a stretch of AGC nucleotides. **b)** An unsealed UPSAS probe consisting out of 4 major parts: a) a target-specific probe sequence with a similar sequence as the reverse primer, b) one or more spacers of at least one nucleotide (may also include an A-stretch as indicated in figure 2a), c) a signal tail that consists out of UGC or TGC nucleotides, and d) a sequence that is complementary to the forward primer used in probe synthesis.
**Figure 3****:** probe synthesis of UPSAS.
   **a)** PCR 1: During the first step, PCR 1 is performed with a forward primer, a reverse primer, a probe template and labeled nucleotides (ATTO, FITC, fast red, biotin or others). The probe template consists out of 5 major parts (from 5' end to 3' end): 1) a sequence that is similar to the forward primer used in probe synthesis and sealing, 2) a part that consists out of AGC nucleotides (template for signal tail), 3) a T-stretch consisting of at least one nucleotide (this is included in the template to stop probe sealing in PCR2). Probe sealing is performed in the absence of dUTP/dTTP, blocking elongation of the seal at the site of the repeat), 4) a spacer of at least one nucleotide, and 5) a sequence that is complementary to the reverse primer and thus to the target-specific probe. After the first PCR run, a single stranded labeled probe is generated of which the reverse primer now constitutes the detection probe part of UPSAS.
   **b)** PCR 2: Optional step to create a 'sealed' probe:
      The second PCR step (PCR 2), is called the "probe sealing" step: During the probe sealing step, primer elongation of the single stranded labeled probe is performed with only one primer (forward primer) in the presence of labeled nucleotides. After PCR2, a partially double stranded probe is generated with the detection probe still free for target recognition and binding. Elongation of forward primer is blocked at the A-repeat at the 3' end of the target-specific probe part because probe sealing is performed with a dNTP-mix consisting out of dATP, dGTP, dCTP and lacking dUTP/dTTP.

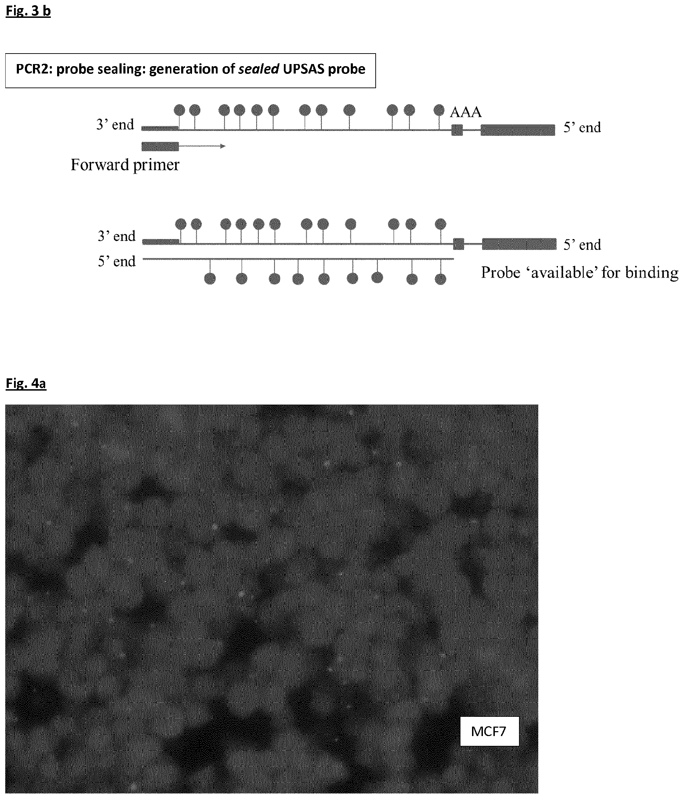

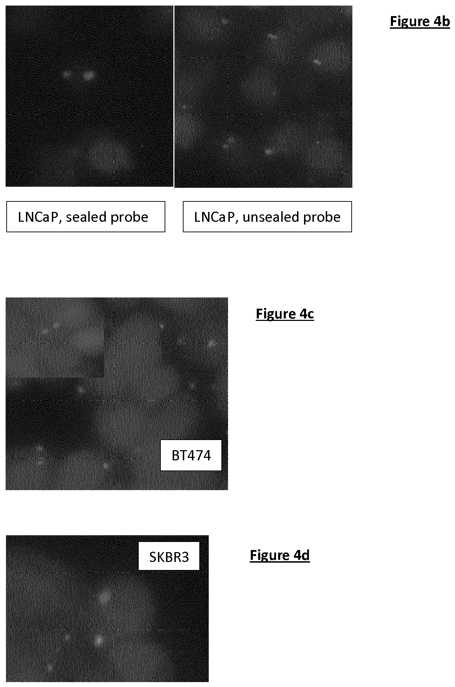

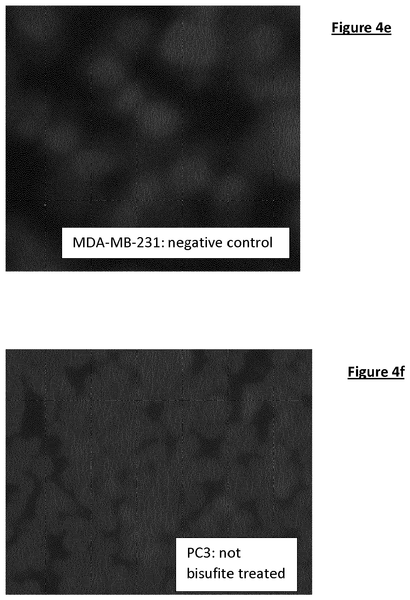

**Figure 4****:** Detection of GSTP1 hypermethylation with UPSAS in cell lines. Fluorescence microscopic evaluation of GSTP1 hypermethylation in MCF7 (Fig 4a), LNCaP (with sealed and unsealed UPSAS probes) (Fig 4b), BT474 (Fig 4c), SKBR3 (Fig 4d), MDA-MD-231 cell lines (Fig 4e) and a PC3 cell line (Fig 4f) not treated with bisulfite. MCF7, LNCaP (with sealed and unsealed UPSAS probe), SKBR3 and BT474 cells show two spots per cell, indicating GSTP1 hypermethylation of both alleles. The MDA-MD-231 cell line and PC3 cell line not treated with bisulfite show no signals, indicating the absence of the GSPT1 hypermethylated target.

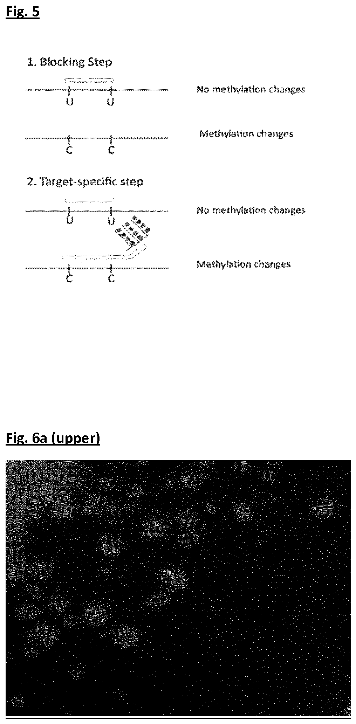

**Figure 5****:** The methylation *in situ* hybridization (MISH) assay. First homologous regions to the target region are blocked by blocking probes. In step 2, target-specific probes are added. Target-specific probes are detected by means of a labeled compound which can be covalently linked to the probe or is linked through hybridization. Blocking and hybridization with the target-specific probes may also be performed in one single step.

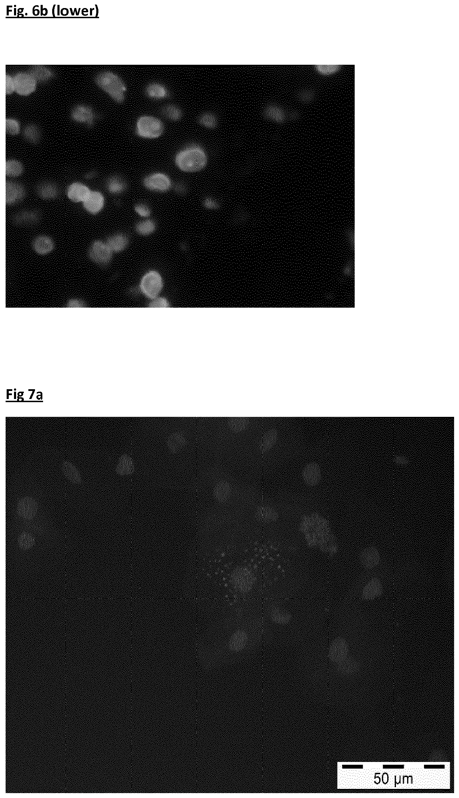

**Figure 6****:** Fluorescence microscopic evaluation of cell adhesion molecule 1 (CADM1 (Overmeer *et al.,* 2008)) hypermethylation in CADM1 positive SiHa cell line (cervical cancer cell line) (Fig. 6b - lower picture) and CADM1 negative skin cells (Fig. 6a - upper picture). SiHa cells show two spots per cells while skin cells do not show any signal.

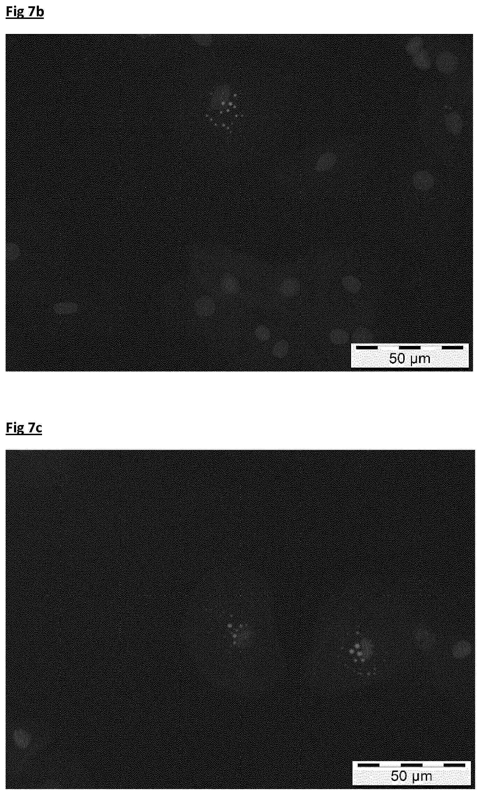

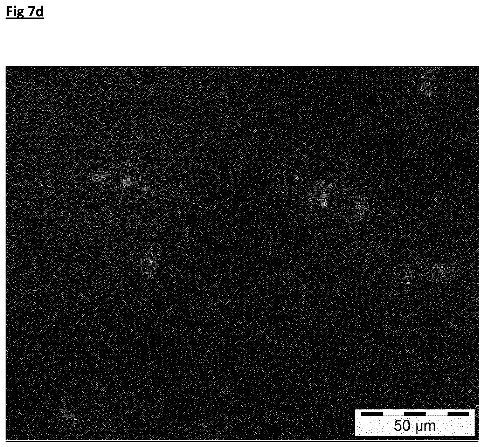

**Figure 7****:** Fluorescence microscopic detection of HPV73 mRNA in monolayers of two HPV73 positive cervical specimens using one L1 (small green dots) and two E1 (larger green dots) specific UPSAS probes (Fig 7a, 7b (specimen 1), 7c and 7d (specimen 2)).

### Summary of invention

The invention relates to
a probe consisting of the following parts indicated in 5' to 3' order: a) a first part comprising a nucleotide sequence of between 14 and 200 nucleotides that is complementary to a target sequence and is similar to a reverse primer, b) a second part functioning as a spacer and comprising at least one nucleotide which is not complementary to the target sequence, c) a third part comprising a nucleotide sequence of between 200 and 100,000 nucleotides which is not complementary to said target sequence, wherein said nucleotides are composed of only 3 different types of nucleotides chosen from the 5 different types A, C, G, T or U and wherein 10 to 100% of said nucleotides are labeled and d) a fourth part comprising a nucleotide sequence which is not complementary to said target sequence but is complementary to a forward primer.

The invention further relates to a probe as defined above further comprising a fifth part comprising the types of nucleotides which are not chosen in the third part.

The invention also relates to a probe as defined above wherein said third part is made double stranded (double stranded probe) by PCR or by hybridization with a (semi-) complementary sequence to the signal tail.

The invention also relates to a process to synthesize a probe as defined above comprising:
a PCR step which is performed in the presence of a reverse primer which is similar to the first part of said probe according to claim 1, a forward primer which is similar to the fourth part of said probe according to claim 1, a mix of labeled and unlabeled dNTPs in order to generate labeling of the probe according to claim 1 and a template consisting of the following parts indicated in the 5' to 3' order: 1) a first part comprising a nucleotide sequence that is similar to a forward primer, 2) a second part comprising a nucleotide sequence of between 200 and 100,000 nucleotides composed of only 3 different types of nucleotides chosen from the 5 different types A, C, G, T or U, 3) optionally, a third part comprising the types of nucleotides which are not chosen in said second part, 4) a fourth part functioning as a spacer and comprising at least one nucleotide, and 5) a fifth part comprising a nucleotide sequence of between 14 and 200 nucleotides that is complementary to a reverse primer.

The invention further relates to a process to synthesize a probe as defined above comprising:
a PCR step according to claim 5 wherein said template comprises said first part and/or third part, and a second PCR step which is performed in the presence of a forward primer, a dNTPs mix which only contain nucleotides which are complementary to the 3 different types of nucleotides chosen in the second part of the template according to the first PCR step.

The invention relates to a process to synthesize probe by hybridization of the signal tail to a complementary sequence to the signal tail.

The invention also relates to the usage of a probe as defined above to specifically detect small target sequences.

The invention further relates to a kit comprising a probe as defined above.

The invention further relates to a method to detect a methylation changes-induced single nucleotide polymorphism *in situ* and/or to distinguish methylation heterogeneity from hemi-methylation and mono-allelic methylation in a sample taken from a patient comprising:
- providing a sample obtained from said patient,
- treating said sample with adequately dosed pepsin and/or protease K and/or HCL and/or detergent and/or ethanol to permeabilize samples and to remove proteins from said sample,
- incubating said sample with adequately dosed bisulfite reagents in the presence of a RNase inhibitor to create non-complementary single stranded DNA strands,
- incubating said samples with specifically designed blocking probes and/or DNA-protecting probes for at least 15 minutes, and
- incubating said sample with specifically designed target-specific probes
wherein said target-specific probes are probes as defined above.

The invention further relates to a kit comprising a probe as defined above.

The invention finally relates to the usage of a kit as defined above to perform the method as defined above.

### Description of invention

The invention consists in first instance of a target-specific amplification probe called the Uniprobe Signal Amplification System (UPSAS) that allows to target and visualize mutations and methylation changes in patients DNA and/or to detect RNA in hybridization assays (dot blot, southern blot, ISH) and its synthesis.

The present invention thus relates to a target-specific amplification probe to detect target RNA and/or DNA sequences in hybridization assays, wherein said probe is characterized by:
its nature as a nucleic acid or nucleic acid analog consisting of a small target-specific detection probe which is able to specifically detect a RNA or DNA target sequences, and is covalently attached to a 'signal tail' which is a nucleic acid or nucleic acid analog of which the sequence only consists out of 3 different types of nucleotides (TGC for example) instead of the usual 4 different types nucleotides (TGCA) and shows no sequence complementarity to the human genome or to the bisulfite converted sequence of the human genome and contains 10% to 100% fluorescent labeled nucleotides.

In other words, the present invention relates to a probe consisting out of 4 major parts : a) a target-specific probe sequence with a similar sequence as the reverse primer, b) one or more spacers of at least one nucleotide (which may also include a stretch of single-type nucleotides such as an A-stretch as indicated in figure 2), c) a signal tail that consists out of 3 different types of nucleotides excluding the type of nucleotide of the latter stretch which is part of said spacer (such as UGC or TGC nucleotides), and d), a sequence that is complementary to a forward primer.

The invention also relates to a method that allows to directly target and visualize methylation changes in single copy genes in patients DNA and RNA *in situ* in one step using the above described specifically designed target-specific probes linked with a labeled compound that contains a vast number of chromogens or fluorescent dyes.

In other words, the present invention provides a non-PCR-based method which directly detects methylation changes in patients DNA or RNA by incorporating a large signal-generating compound (labeled compound or signal tail) at the target-specific part of the target-specific probes rather than replicating target sequences for sufficient detection. Therefore, the present invention allows for detection of small targets including methylation changes at physiological levels.

The present invention thus relates to said 'target-specific probes' characterized by:
their nature as a nucleic acids or nucleic acid analog, consisting of a small target-specific part, which is able to distinguish nucleotide polymorphisms and methylation changes, and is bound to one or more labeled compound(s), which is a nucleic acids or nucleic acid analog of which the sequence(s) shows no sequence complementarity to the human genome or to the bisulfite converted sequence of the human genome and contains 10% to 100% fluorescent labeled nucleotides.

More specifically, UPSAS is characterized by:
a. Its nature as a nucleic acid or nucleic acid analog,
b. Its comprehensive structure comprising:
   1. An target-specific detection probe sequence at 5' end.
   2. At the 3' end of the sequence of said detection probe part, one or more spacers are included that ensures that the signal amplification part does not sterically interferes with probe binding. One of the spacers may also include a stretch of single-type nucleotides, comprising the types of nucleotides that is excluded in the signal tail.
   3. At the 3' end of said spacers, a signal tail between between 200 bp and 100 kbp is included. The signal tail contains 10% to 100% fluorescent labeled nucleotides.
   4. At the 3' end of the signal tail a forward primer recognition site is included. The primer recognition site is used as a primer annealing site during probe sealing (the signal tail is made double stranded in a second PCR reaction to create sealed UPSAS; probe sealing is performed to reduce specific binding and/or for incorporation of additional labels) but it also used in the first PCR reaction for probe synthesis (to aim an exponential increase of the amount of probe).
   5. Complementary (only for sealed UPSAS) to the signal tail a labeled or unlabeled nucleotide strand ("seal") may be included. By making the signal tail double stranded, aspecific binding of the signal tail and thus mistargeting of the probe is avoided.

The target-specific detection probe part of UPSAS is characterized by:
1. Its code: it should be) complementary to the sequence of the target-sequences, through Watson-Crick base pairing. For example: if the sequence of the methylation marker for a specific disease is 5' -GTT GTG TAA TTC GTT GGA TGC GGA TTA GGG CG - 3' (SEQ ID N° 15), ideally the target-specific probes for the sense and anti-sense strand should respectively be 5'-CGC CCT AAT CCG CAT CCA ACG AAT TAC ACA AC-'3 (SEQ ID N° 16).
2. Their size: the target-specific part of the probe is between 14-200 bp

The 'signal tail' is specifically characterized by:
1. Its nature as a nucleic acid or nucleic acid analog
2. Its structure:
   a. Its size: the signal tail is between 200bp and 100 kbp.
   b. The signal tail consists out of 10-100% bases modified with chromogens or fluorescent dyes (for instance ATTO, biotin, FITC, Alexa Red and/or others).
   c. For sealed UPSAS: the signal tail is (partially) made double stranded with a "seal" to prevent aspecific binding of UPSAS. Hereupon, the seal may also contain labeled nucleotides and may thus be used to incorporate additional labels in the probe.
3. Its code: remaining aspecific binding of the signal tail is prevented by the nature of its sequence composition: the signal tail mainly consists out of 3 nucleotides (for example U/TGC if one spacer consists out of a 'stretch' of A nucleotides, or, U/TGA if one spacer consists out of a 'stretch' of C nucleotides, or, U/TAC if one spacer consist out of a 'stretch' of G nucleotides, or, AGC, if one spacer consists out of a 'stretch' of T nucleotides) instead of the 'usual' 4 nucleotides (TGCA).

The "spacers" between the signal tail and the detection probes are specifically characterized by:
1. Its nature as a nucleic acid or nucleic acid analog
2. Its structure (figure 2a):
   a. For sealed UPSAS:
      i. The first spacer next to the signal tail is for example an adenine-repeat. It is composed of a stretch of 1 - 10 000 and preferentially of 1 - 200 A, C, G, T or U.
      ii. Optionally, additionally spacers of 1 - 10 000 and preferentially of 1 - 200 nucleotides are included next to the A-stretch. The sequences of these spacers show no complementary to the sequences flanking the target region in order to prevent potential interference of the spacers with probe binding.
   b. For unsealed UPSAS:
      i. Spacers of 1 - 10 000 and preferentially of 1-200 nucleotides are included next to the target-specific probe part. The sequences of these spacers show no complementary to the sequences flanking the target region in order to prevent potential interference of the spacers with probe binding.

An annealing site for the forward primer is included at at the 3'end of the UPSAS probe. The forward primer annealing site is characterized by:
a. Its nature as a nucleic acid or nucleic acid analog
b. Its size: between 8bp and 10 kbp and preferentially between 14-200 p
c. Its complementary to the forward primer used for probe synthesis and probe sealing

The forward primer annealing site is mandatory for the synthesis of sealed UPSAS in case the signal tail is made double stranded by PCR to reduce aspecific binding and/or for incorporation of additional labels but optional for the synthesis of unsealed UPSAS or in case the sealed probe is made double stranded by hybridization with the seal.

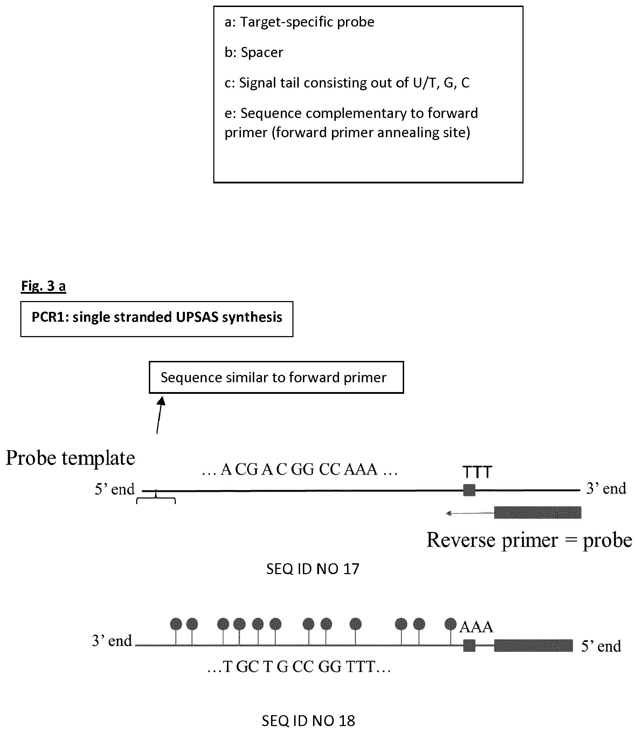

Synthesis of UPSAS is performed in one (unsealed UPSAS) or two PCR steps (sealed UPSAS) (Figure 3):
a. During the first step, PCR is performed with a forward primer (for unsealed UPSAS or when sealed UPSAS is created by hybridization with the seal, mandatory for sealed UPSAS created by PCR), a reverse primer (the reverse primer will constitute the target detection probe), a probe template and labeled nucleotides (ATTO, FITC, fast red, biotin and/or other labels). After the first PCR a single stranded labeled probe is generated.
b. The second PCR step, is called the "probe sealing" step (only required for PCR-sealed UPSAS synthesis):
   During the probe sealing step, primer elongation is performed with only one primer (forward primer) in the presence of labeled nucleotides. After PCR2, a partially double stranded probe is generated with the detection probe still free for target recognition and binding. Elongation of forward primer is blocked at the A-repeat at the 3' end of the target-specific probe part because probe sealing is performed with a dNTP-mix consisting out of A, G, C and lacking U/T.

Synthesis of sealed UPSAS may also be performed by hybridization of unsealed UPSAS with a complementary sequence (seal) of the signal tail.

Hence, the probe (UPSAS) of the present invention has a double function:
a. Specific detection of small target sequences with the target-specific probe part: longer sequences may be detected using a set of probes binding next to each other, and
b. Direct signal amplification of the part containing the target-specific probe: visualization of the target-specific probe(s).

The present invention also relates to a method to detect methylation changes and/or to distinguish methylation heterogeneity from hemi-methylation and mono-allelic methylation *in situ* in a sample taken from a patient comprising:
- providing a sample obtained from said patient,
- treating said sample with adequately dosed pepsin and/or protease K and/or HCL and/or detergent and/or ethanol for permeabilization of cells and/or to remove proteins from said sample,
- incubating said sample with adequately dosed bisulfite reagents in the presence of a RNase inhibitor to create non-complementary single stranded DNA strands,
- incubating said samples with specifically designed 'blocking probes' and/or 'RNA- or DNA-protecting probes' for at least one 1 hour, and
- incubating said sample with specifically designed 'target-specific probes' as specified above.

With the term 'methylation changes' is meant the conversion of unmethylated cytosines into methylated cytosines and *vice versa.*

With the term 'heterogeneity' is meant a heterogenic pattern of unmethylated and methylated target genes in the same cell/sample.

With the term 'hemi-methylation' is meant methylation changes that occur at one of the two DNA strands of one allele.

With the term 'mono-allelic methylation' is meant methylation changes that occur at one of the two alleles per gene.

With the term 'a sample of a patient' is meant a section cut from a FFPE tissue block, a fresh frozen tissue, a cell monolayer, or a smear acquired from a patient.

With the terms 'adequately dosed pepsin and/or protease K and/or HCL and/or detergent' is meant the optimal amounts of pepsin or proteinase K provided, ranging from 0.001% to 10% pepsin/proteinase K, the optimal amount of HCL provided ranging from 0.005 M HCL to 4 M HCL, an optimal amount of detergent (Triton X-100, Tween-20) provided ranging between 0.01% and 4 %.

With the terms 'adequately dosed bisulfite reagents in the presence of a RNase inhibitor' is meant the optimal provided concentrations of bisulfite, NaOH and RNase inhibitor ranging from 1 to 8 M bisulfite, 0.1 M to 1 M NaOH and 1:3 to 1:10000, preferentially between 1:100 to 1:1000 RNase inhibitor, respectively.

The present invention relates to said 'target-specific probes' to detect methylation changes characterized by:
their nature as a nucleic acids or nucleic acid analog, consisting of a small target-specific part, which is able to distinguish nucleotide polymorphisms and methylation changes, and is bound to one or more labeled compound(s), which is a nucleic acids or nucleic acid analog of which the sequence(s) shows no sequence complementarity to the human genome or to the bisulfite converted sequence of the human genome and contains 10% to 100% fluorescent labeled nucleotides.

More specifically, 'target-specific probes' are characterized by:
a. Their nature as a nucleic acids or nucleic acid analog, consisting out of a target-specific part and a labeled compound
b. Their number: a minimum of 2 target-specific probes targeting the sense and/or anti-sense strand are included, in order to increase test sensitivity.
c. The target-specific part is specifically characterized by:
   1. Their code: it should be complementary to the bisulfite converted target region in the DNA and/or RNA, containing a high frequency of CG sequences, through Watson-Crick base pairing. These short stretches of DNA in which the frequency of the CG sequence is higher than other regions are called CpG islands. CpG islands are often located around the promoters of genes. The promotor region of a gene is localized -2000 to +500, or more strict this can be limited to -500 to + 200, of the transcription start site (TSS) of the gene. A specific methylation pattern of these CpG islands in the promoter region that is characteristic for a disease state is called a methylation marker. A methylation pattern is defined as the order in which the methylated or unmethylated C in the CpG islands is present or absent in a target sequence. Bisulfite treatment induces unmethylated cytosine to be converted into uracil but leaves 5' methylcytosine residues unaffected. For example: after bisulfite treatment the methylated sequence 5'-ACmGTCCATCmGCT3'- (SEQ ID N° 3) will be converted into 5'-ACmGTUUATCmGUT-3' (SEQ ID N° 4). The unmethylated counterpart sequence 5'-ACGTCCATCGCT-'3 (SEQ ID N° 5) will be converted in 5'-AUGTUUATUGUT-'3 (SEQ ID N° 6). The target-specific probe will specifically bind methylation markers in genes that should be detected, after bisulfite treatment. For example: if the sequence of the methylation marker for a specific disease in the anti-sense strand is 5'-GAGG**CmG**C**CmG**C**CmG**C**CmG**C**CmG**CTGC**CmG**C**CmG**CACACTGGGATC**CmG**CT **CmG**GCAGCA-3' (SEQ ID N° 7), ideally the target-specific probes for the sense and anti-sense strand should respectively be 5'-**C G**AA **CG**A ATC CCA ATA TA**C G**A**C G**AC AA**C G**A**C G**A**C G**A**C G**A**C G**C-'3 (SEQ ID N° 8) and 5'-**CG**C **CG**C **CG**C **CG**C **CG**C TAC **CG**C **CG**C ACA CTA AAA TC**C G**CT **CG**A-'3 (SEQ ID N° 9).
   2. Their size: the target-specific part of the probe is designed in such a way that it can extend between one and 10 base pairs beyond the last CpG of the intended targeted region, in order to maximize temperature difference between target specific sequences and the mismatched sequence in a particular hybridization buffer, leading to specific hybridization to the template DNA. Preferably, the probe extents one base pair beyond the last CpG of the target region.
   3. Their linkage to 'a labeled compound': the target-specific part is covalently attached to (a) labeled compound(s) (figure 1). The linkage between the target-specific part and (the) labeled compound(s) is established by a linker. The linker is an 1 to 20 bp long nucleotide sequence that shows no complementarity to the bisulfite converted sequences of the methylation marker 5' or 3' flanking regions, depending at which end the linker is placed, since this may interfere with mismatch detection. The linker may also be a spacer (for instance a glycol spacer).
d. The 'labeled compound' is characterized by:
   1. Its nature as a nucleic acid or nucleic acid analog
   2. Its direct attachment to the target-specific compound (figure 1): the labeled complex is covalently linked to the target-specific compound by means of a linker. The linker is a nucleotide sequence of 1 to 20 bp or is a spacer (for instance glycol spacers).
   3. Its structure:
      a. The labeled compound is large: between 200 bp and 100 kbp.
      b. The labeled compound consists out of bases modified with chromogens or fluorescent dyes (for instance biotin, FITC, Alexa Red and others).
      c. In case blocking of the labeled compound is pursued, a primer annealing sequence for PCR amplification is included at the 3'end in the labeled sequence. At the 5' end of the labeled compound, a single nucleotide may be incorporated that shows complementarity to a particular dideoxy nucleoside triphosphate (ddNTP), which is included in PCR step 2. Adding a specific ddNTP that is complementary to a nucleotide, specifically present at the 3' end of the linker, ensures that the labeled compound and not the target-specific part is sealed during PCR step 2.
      d. The labeled compound can be sealed by a complementary sequence to prevent aspecific binding (figure 1).
   4. Its sequence: the labeled compound has no or a low complementarity to the human genome and the bisulfite converted human genome.

The blocking probes as described further are characterized by:
their nature as a unlabeled nucleic acid or nucleic acid analog, having a complementary sequence to similar sequences as the target sequence for instance the bisulfite converted unmethylated counterpart sequence of the target region and having a maximal bp difference of 1 kbp and preferentially of 40 bp to the target-specific part of the target-specific probes used in the same assay.

More specifically, the present invention relates to said blocking probes characterized by:
a. Their nature as a nucleic acid or nucleic acid analog
b. Their number: A minimum of 1 and preferentially of 2 blocking probes blocking the sense and/or anti-sense strand are included, in order to increase test specificity.
c. Their code: it should be complementary (by means of Watson-Crick base pairing) to generic sequences, to similar sequences as the target sequence and in case methylation changes are detected, to bisulfite converted sequences of the DNA and/or RNA. More specifically, it blocks generic sequences, similar sequences to the target-sequence(s) and/or the bisulfite converted unmethylated sequence(s) at the target region (in case the target-specific probe should detect the methylated sequence). Blocking probes thus block non-specific binding of target-specific probes to competing DNA/RNA sequences by binding these sequences. As a consequence, the target-specific probe will have the opportunity to specifically bind the target region in methylation markers in genes that should be detected (after bisulfite treatment). For examples: if the sequence of a methylation marker for a disease in the anti-sense strand is 5'-GAGG**CmG**C**CmG**C**CmG**C**CmG**C**CmG**CTGC**CmG**C**CmG**CACACTGGGATC**CmG**CT**CmG**GC AGCA-'3 (SEQ ID N° 10), ideally the blocking probes for the sense and anti-sense strand should respectively be 5'-C**A**A AC**A** AAT CCC AAT ATA C**A**A C**A**A CAA C**A**A C**A**A C**A**A C**A**A C**A**C-'3 (SEQ ID N° 11) and 5'-C**A**CC**A**CC**A**CC**A**CCACT**A**CC**A**CCACACACTAAA ATCC**A**CTC**A**A-'3 (SEQ ID N° 12).
d. Their size: The blocking probes used in methylation assays are designed in such a way that they can extend between one and 10 bp and ideally between one and 2 bp beyond the last CpG of the intended targeted region. Blocking probes have the same length or a maximal size difference of 1 kbp and preferentially of 4 bp as the target-specific probes used in the same assay. The blocking probes are between 8 bp and 10 kbp and ideally between 14 - 200 bp long.
e. Their distinctive character to discriminate the unmethylated target from the methylated target in methylation assays: its distinctive character to discriminate a methylated target from an unmethylated target is superior to this of the target-specific probe used in the same assay.
e. Their freedom from labeling with chromogens or fluorescent dyes: the blocking probes are unlabeled probes.

The unlabeled DNA protecting probes as described further are characterized by:
their nature as a unlabeled nucleic acid or nucleic acid analog, showing (semi-) complementarity to the sequences flanking the target sequences at the 5'end and/or 3'end in the DNA and/or RNA, through Watson-Crick base pairing and are used to 'relax' the target sequences and enhance binding of the blocking and/or target-specific probes.

More specifically, the present invention relates to the latter unlabeled DNA protecting probes characterized by:
a. Their nature as a nucleic acid or nucleic acid analog
b. Their number: a minimum of 1 protecting probe is included, in order to hybridize to (a) sequence(s) flanking the target sequence(s) at the 5'end and/or 3'end
c. Their code: they should be (semi-) complementary to the sequences flanking the (bisulfite converted) target region at the 5'end and/or 3'end in the DNA and/or RNA, through Watson-Crick base pairing. More specifically, they should be (semi-) complementary to the sequence 1 to 50 bp adjacent to the 5' end or 3'end of the target region. They relax the RNA and (bisulfite converted) DNA regions by forming a double stranded structure, when they bind to the sequences flanking the target region(s). Consequently, the target region is put in a favorable position for binding the blocking probes or target-specific probes. The DNA-protecting probes will specifically bind the sequences adjacent to the target regions; in methylation assays this is around the methylation markers in genes that should be detected (after bisulfite treatment).
d. Their size: The DNA protecting probes are between 10 and 100 kbp and ideally between 40 bp and 1 kbp. For methylation assays they are designed in such a way that the 5'end or 3'end of the probe is between 1 and 20 base pairs beyond the last CpG of the intended targeted region. For example, if the sequence flanking the bisulfite converted target sequence at the 5'end is 5'-TGG TTA AGG TTA TTG GGG TGT TTT TGG AGA TTT AGG GGT TAA TTG GTT GGT GTT TAT ATT TAT TTG TGG GGA TTA GTG TTG TGG TGG AGA AGA GTA ATA GTA GAA GTT GGA GTT GGA GTT TGG GAG-'3 (SEQ ID N° 13); ideally the 5' sense protecting probe should be: 5'-C RAC TCC RAC TTC TAC TAT TAC TCT TCT CCR CCR CRA CAC TAA TCC CCA CAA ATA AAT ATA AAC ACC RAC CRA TTA ACC CCT AAA TCT CCR AAA ACA CCC CAA TAA CCT TAA CCA-3'(SEQ ID N° 14), with R= Adenosine or guanine.
e. Their freedom from labeling with chromogens or fluorescent dyes.

The present invention also relates to a kit comprising UPSAS as defined above.

The present invention relates to the usage of the latter kit to perform the method as described above.

The term 'kit' refers to any manufacture (e.g. a package or a container) comprising at least one reagent/probe as described above for performing an assay/method as described above. Positive and/or negative controls can be included in the kits to validate the activity and correct usage of reagents employed in accordance with the present invention. The design and use of controls is standard and well within the routine capabilities of those of ordinary skill in the art. The kit can be promoted, distributed, or sold as a unit for performing the methods or usages of the present invention.

Additionally, the kits can contain a package insert describing the kit and methods/usages for its use. The term 'kit' is for example also described in WO 2009/141359.

The present invention will now be illustrated by the following, non-limiting examples.

### Examples

### Example 1: Synthesis of the UniProbe Signal Amplification System (UPSAS) probe

Four different UPSAS probes corresponding to the Glutathione S-Transferase Pi 1 (GSTP1), hypermethylated regions in prostate cancer were designed and synthesized by PCR.

The 4 templates used for probe synthesis consist out of 5 major parts (from 5' end to 3' end): 1) a sequence that is similar to the forward primer used in probe synthesis and sealing, 2) a part that consists out of AGC nucleotides (template for signal tail), 3) a T-stretch of three nucleotides (this is included to stop probe sealing when probe sealing is performed with three nucleotides), 4) a spacer of 9 nucleotides, and 5) a sequence that is complementary to the reverse primer and thus to the target-specific probe.

During the first PCR step, a forward primer (FP) with the same sequence as the sequence found at the 5'end of the probe template and a reverse primer (RP) that will form the target-specific probe part of UPSAS, were used for amplification. An PCR reaction mix containing the forward primer, reverse primer, unlabeled dCTPs, dGTP and dATP, labeled dUTPs, unlabeled dUTP, Taq DNA Polymerase, PCR buffer, MgCI2, nuclease-free H2O and template, was made. Optionally, a second PCR step was be performed to (partially) seal the signal tail with a complementary sequence ("seal").

Only the FP is used for the second PCR step; the primer is complementary to a sequence at the 3' end of the probe. An PCR reaction mix was made containing the forward primer, unlabeled dCTPs, unlabeled dGTP, unlabeled dATP, labeled dATP, Taq DNA Polymerase, PCR buffer, MgCl2 and nuclease-free H2O. PCR2 was performed in the presence of only three nucleotides to stop elongation before the target-specific probe part, which is kept available for target binding. After the second PCR step a target-specific probe, of which the signal tail carries a complementary sequence ("seal") and thus prevents aspecific binding of the signal tail and carries additional signals, is generated.

Sealing of the unsealed probe may also be performed by incubating the unsealed probe with the "seal" allowing hybridization of the signal tail with the seal.

### Example 2: Probe labeling is performed efficiently

Probes were run on gel to confirm probe labeling and to estimate the amount of labels per probe; The probes contained at least 250 labels after PCR1 and 500 labels after PCR2.

Biotin-labeled GSTP1 probes were spotted on a nylon membrane prior to staining with 3,3'-Diaminobenzidine (DAB): spots stained dark brown, indicating that labels were not only incorporated in the probes as could be observed based on the molecular weight size on gel, but also gave strong signals.

### Example 3: Tissue morphology is kept intact after ISH pretreatment steps combined with bisulfite conversion in situ.

3 µM formalin-fixed and paraffin-embedded (FFPE) cervical tissue sections were cut and stretched on the glass slide and deparaffinized in xylene. The sections were dehydrated 2 times for 5 minutes in 100% ethanol. Hereupon, the sections were incubated in 0.2 N HCI and washed with ultrapure water. The sections were treated for 28 minutes at 37°C with porcine pepsin and washed 2 times for 5 minutes with ultrapure water. The sections were treated for 15 minutes with 0.1% triton and washed for 3 minutes with molecular grade water afterwards. Subsequently, sections were incubated with 150 µl of a bisulfite mix (Zymo) for 4 hours. After a 15 minute desulfonation step, the samples were washed with molecular grade water and stained with haematoxylin and eosin (H&E) to evaluate conservation of the tissue morphology. Tissue morphology was evaluated by an experienced, university level pathologist, who confirmed that the morphology was kept intact.

This experiment states that bisulfite treatment *in situ* and the necessary pretreatment that should be performed prior to probe hybridization do not interfere with tissue morphology.

### Example 4: Bisulfite treatment generates single stranded DNA (ssDNA) in situ.

We compared DAPI staining between samples incubated with bisulfite for 1, 2, 3 and 4 hours. DAPI gives brighter signals when binding to double stranded DNA than when binding to single stranded DNA. As a control we included a FFPE tissue slide that was not pretreated with bisulfite.

3 µM FFPE cervical tissue sections were cut and deparaffinized in xylene. The slides were dehydrated 2 times for 5 minutes in 100% ethanol. Hereupon, slides were incubated with RNase A (100µg/ml RNase A in 2X Saline Sodium Citrate (SSC) for 45 min at room temperature (RT) and they were washed with 2xSSC (2x5 minutes). Slides were then treated with 0.2 M HCI and washed with ultrapure water. The slides were treated for 28 minutes at 37°C with porcine pepsin and washed 2 times for 5 minutes with ultrapure water. The slides were treated for 15 minutes with 0.1% triton and washed for 3 minutes with molecular grade water afterwards. Subsequently, sections were incubated with 150 µl of a bisulfite solution (Zymo) for 1, 2, 3 and 4 hours. After a 15 minute desulfonation step, the slides were stained with DAPI.

Samples with a four hour bisulfite incubation showed the highest reduction in DAPI brightness, indicating that the most ssDNA is generated after a four hour bisulfite treatment.

### Example 5: In samples pretreated according to the described protocol, in situ bisulfite conversion is an efficient process.

ISH pretreatment and bisulfite conversion was performed on 9 FFPE skin sections; on one set of samples an additional HCL step was performed.

7 µM FFPE skin sections were cut and deparaffinized in xylene. Slides were dehydrated 2 times for 5 minutes in 100% ethanol. Hereupon, the slides were incubated in 0.2 M HCI and washed with ultrapure water and 2xSSC. The slides were then incubated for 37 minutes in 1 M NaSCN (VWR) at 80°C and washed with ultrapure water and 2x SSC. The slides were treated for 28 minutes at 37°C with porcine pepsin and washed 2 times for 5 minutes with 2xSSC. Slides were treated for 15 minutes with 0.1% triton and washed for 3 minutes with molecular grade water afterwards. Subsequently, sections were incubated with 150 µl of a bisulfite solution (Zymo) for 4 hours. After a 15 minute desulfonation step, the samples were washed with molecular grade water and they were scraped off in a reaction tube containing Trizol. The samples were homogenized using a tissue mixer and bisulfite converted DNA was purified. Picogreen and ribogreen were used to respectively measure ds- and ssDNA concentrations in the sample extracts. The concentration of the single stranded product significantly increased with an increasing incubation time. PCR amplification with primers specific for the bisulfite converted regions of two genes (ACTB and TWIST (Renard *et al.,* 2010)) was performed; efficient qPCR amplification occurred in all cases: a higher Ct (cycle threshold) value was seen in samples treated with HCL. To confirm efficient bisulfite conversion a set of samples (incubated with bisulfite for 4 hours) was sequenced. All samples showed 99,93 to 99,97% bisulfite conversion in the amplified regions, indicating that *in situ* bisulfite conversion was optimal in these samples.

### Example 6: UPSAS probes detect methylation changes in FFPE cell lines

Fluorescent labeled UPSAS probes were synthesized and used to detect GSTP1 hypermethylation in breast and prostate cancer cell lines.

MCF7, LNCaP, SKBR3, BT474, PC3 and MDA-MB-231 cell lines were first tested for GSTP1 hypermethylation by MSP; In all cell lines except for MDA-MB-231 hypermethylated GSTP1 copies were detected. The amount of hypermethylated GSTP1 copies ranged between 9.7% for LNCaP and 47,1% for PC3. MDA-MB-231 was therefore used as a negative control for GSTP1 hypermethylation.

4 micron FFPE MCF7, LNCaP, SKBR3, BT474, PC3 and MDA-MB-231 slides were cut and deparaffinized in xylene. Slides were dehydrated in 100% ethanol. Hereupon, the slides were incubated with HCL. The slides were then washed with ultrapure water and 2xSSC. Slides were treated with porcine pepsin and washed with 2xSSC. Slides were incubated with a bisulfite solution for 3 hours at 54°C, followed by a washing step with 2xSSC. After an 15 minutes desulfonation step, the slides were washed with 2xSSC. The PC3 cell line section was not treated with bisulfite but only incubated with molecular grade water. The slides were dehydrated using ethanol series (70%, 90% and 100%). After air-drying, slides were incubated overnight at 42°C with GSTP1 methylation-specific probes. LNCaP slides were incubated with sealed or unsealed probe. Post-hybridization washes were performed in 2xSSC, 0.1xSSC and mounted for microscopic evaluation. In the majority of the cells two dots were observed, indicating GSTP1 hypermethylation of both alleles (Figure 3).

### Example 7: Generation of methylation target-specific probes for CADM1

Target-specific probes consist out of two compartments: 1) at one end an unlabeled target-specific part that recognizes the methylation changes (in this case CADM1 hypermethylation (Eijsink *et al.,* 2012; Overmeer *et al.,* 2008), and 2) at the other end a labeled compartment whose function is to allow signalization of the target-specific part. Both compartments form the target-specific probe. The target-specific probes are synthesized by PCR. The template that is used for the generation of the probe consists of two core sequences: 1) One sequence that is identical to the target region, and 2) one random sequence of 200 bp to 100 kbp that shows low or no sequence similarity to the human genome or to the bisulfite converted sequence of the human genome. One or two PCR steps are used for generation of the target-specific probes. During the first PCR step, an primer that is complementary to the target-specific region is used for amplification. A reaction mix containing 500 nM primer, 100 µM unlabeled dNTPs, 100 µM labeled dNTPs, 0.3 µl Taq DNA Polymerase, PCR buffer 2 µl, 1 ng template and nuclease-free H2O was made. PCR cycling conditions are 1) Denaturation 98°C - 1 min, 2) Cycling 1: 98°C - 20 sec, 64°C (sense) or 72°C (anti-sense) - 45 sec, 72°C, 5 min, 20 repeats, 3) Final extension: 72°C - 1 min, 4) Hold: 4°C, ∞. After the first PCR step, target-specific probes are generated. Optionally, a second PCR step may be performed to seal the labeled sequence with a complementary sequence. First, the probe concentration generated probe during "PCR step 1" is measured with ribogreen and 1 ng of the generated probe is used as an input for the second PCR step. One primer is used for the second PCR step, the primer is complementary to a 18 to 100 bp region at the 3' end of the target-specific probe. A 20 µl reaction mix was made containing 500 nM primer, 200 µM unlabeled dNTPs, 0,5 µM of a ddNTP, 0.3 µl Taq DNA Polymerase, 2 µl PCR buffer, 1 ng template and nuclease-free H2O. The PCR conditions used were: 1) Denaturation 98°C - 1 min, 2) Cycling 1: 98°C - 20 sec, 60°C - 45 sec, 72°C min, 20 repeats, 3) Final extension: 72°C -1 min, 4) Hold: 4°C, ∞. ddNTPs are used to stop elongation before the target-specific part, which is kept available for target binding. After the second PCR step a target-specific probe, of which the labeled component carries a complementary sequence that seals the labeled compound and thus prevents aspecific binding of the labeled compound to patients DNA, is recovered.

### Example 8: Methylation In situ hybridization assay (MISH) for CADM1 hypermethylation

MISH was demonstrated in FFPE SiHa cell lines (cervical cancer cell line). SiHa cell lines are characterized by CADM1 gene hypermethylation, a potential biomarker for squamous cervical cancer (SCC).

Blocking probes, specific for unmethylated sequences, target-specific probes, including a labeled compound and DNA protecting probes, are designed for the CADM1 gene. These probes can be designed for any gene. 3 µM FFPE SiHa cell line sections and skin sections were cut and deparaffinized in xylene. Slides were dehydrated 2 times for 5 minutes in 100% ethanol. Hereupon, the slides were incubated with RNase A (100µg/ml RNase A in 2XSSC) for 45 min at room temperature (RT) and washed with 2xSSC (2x5 minutes). The slides were then incubated in HCI and washed with ultrapure water and 2x SSC. Slides were treated for 37 minutes with 1 M NaSCN (VWR) at 80°C and washed for 1 minute with ultrapure water and 2 times for 5 minutes with 2xSSC. Slides were treated for 28 minutes at 37°C with porcine pepsin and washed 2 times for 5 minutes with 2xSSC. Slides were incubated for 5 minutes with 50% formamide at 95°C. Excessive formamide was removed and the sections were incubated with an 150 µl bisulfite solution for 4 hours at 50°C. After an 15 minutes desulfonation step, the slides were washed 2 times for 5 minutes with 2xSSC and post-fixed with 1% formaldehyde. The slides were dehydrated using an ethanol series (70%, 90% and 100%). After air-drying, slides were incubated with blocking-probes and DNA protecting probes for 3 hours at 45°C. Post-hybridization washes were performed in 2XSSC/0,3% NP-40 at room temperature (RT), at 42°C to 72°C and at RT. A second incubation with CADM1 methylation-specific probes (6nM) was performed overnight at 45°C. Post-hybridization washes were performed in 2xSSC at RT, at 42°C to 72°C and at RT. The slides were then incubated with Streptavidin-FITC (1:500). Unbound streptavidin was washed away with 2xSSC. Slides were counterstained with DAPI. Consequently, hypermethylated CADM1 was evaluated *in situ* with a fluorescence microscope. In defined conditions, SiHa cells showed predominantly two noticeable signals per cell and skin tissue cells did not show any signal, implying specific binding of CADM1 target probes (Figure 6).

### Example 9: UPSAS probes are able to specifically detect and visualize single HPV73 mRNA transcripts in cervical monolayers

A set of 3 HPV73 UPSAS probes recognizing HPV73 L1 and E1 mRNA were designed and synthesized.

HPV73 cervical smears confirmed by qPCR using HPV73 specific primers were analyzed with the synthesized HPV73 UPSAS probes. Monolayers were fixated with paraformaldehyde (PFA), permeabilized with proteinase K and incubated with a hybridization mixture containing 3 HPV73 mRNA detecting probes. Monolayers were washed and visualized with the fluorescence microscope. Individual HPV73 L1 and E1 mRNA were adequately detected with the 40x objective (figure 7).

### References

Collins ML, Irvine B, Tyner D, Fine E, Zayati C, Chang C, Horn T, Ahle D, Detmer J, Shen LP, Kolberg J, Bushnell S, Urdea MS, Ho DD. A branched DNA signal amplification assay for quantification of nucleic acid targets below 100 molecules/ml. Nucleic Acids Res. 1997;25(15):2979-84.
Eijsink, J.J.H., LENDVAI, A., Deregowski, V., Klip, H.G., Verpooten, G., Dehaspe, L., de Bock, G.H., Hollema, H., Van Criekinge, W., SCHUURING, E., et al. (2012). A four-gene methylation marker panel as triage test in high-risk human papillomavirus positive patients. Int. J. Cancer 130, 1861-1869.
Larsson, C., Koch, J., Nygren, A., Janssen, G., Raap, A.K., Landegren, U., and Nilsson, M. (2004). In situ genotyping individual DNA molecules by target-primed rolling-circle amplification of padlock probes. Nat Meth 1, 227-232.
Li, Y., Miyanari, Y., Shirane, K., Nitta, H., Kubota, T., Ohashi, H., Okamoto, A., and Sasaki, H. (2013). Sequence-specific microscopic visualization of DNA methylation status at satellite repeats in individual cell nuclei and chromosomes. Nucleic Acids Research 41, e186-e186.
Nuovo, G.J., Plaia, T.W., Belinsky, S.A., Baylin, S.B., and Herman, J.G. (1999). In situ detection of the hypermethylation-induced inactivation of the p16 gene as an early event in oncogenesis. Proc. Natl. Acad. Sci. U.S.a. 96, 12754-12759.
Overmeer, R.M., Henken, F.E., Snijders, P.J.F., Claassen Kramer, D., Berkhof, J., Helmerhorst, T.J.M., Heideman, D.A.M., Wilting, S.M., Murakami, Y., Ito, A., et al. (2008). Association between dense CADM1 promoter methylation and reduced protein expression in high-grade CIN and cervical SCC. J. Pathol. 215, 388-397.
Renard, I., Joniau, S., van Cleynenbreugel, B., Collette, C., Naômé, C., Vlassenbroeck, I., Nicolas, H., de Leval, J., Straub, J., Van Criekinge, W., et al. (2010). Identification and Validation of the Methylated TWIST1 and NID2 Genes through Real-Time Methylation-Specific Polymerase Chain Reaction Assays for the Noninvasive Detection of Primary Bladder Cancer in Urine Samples. European Urology 58, 96-104.
Schriml LM, Padilla-Nash HM, Coleman A, Moen P, Nash WG, Menninger J, Jones G, Ried T, Dean M. Tyramide signal amplification (TSA)-FISH applied to mapping PCR-labeled probes less than 1 kb in size. Biotechniques. 1999;27(3):608-13.

## Claims

1. A probe consisting of the following parts indicated in 5' to 3' order: a) a first part comprising a nucleotide sequence of between 14 and 200 nucleotides that is complementary to a target sequence and is similar to a reverse primer, b) a second part functioning as a spacer and comprising at least one nucleotide which is not complementary to the target sequence, c) a third part comprising a nucleotide sequence of between 200 and 100,000 nucleotides which is not complementary to said target sequence, wherein said nucleotides are composed of only 3 different types of nucleotides chosen from the 5 different types A, C, G, T or U and wherein 10 to 100% of said nucleotides are labeled and d) a fourth part comprising a nucleotide sequence which is not complementary to said target sequence but is complementary to a forward primer.

2. A probe according to claim 1 further comprising a fifth part comprising the types of nucleotides which are not chosen in the third part.

3. A probe according to any of claims 1-2 wherein said third part is made double stranded by PCR.

4. A probe according to any of claims 1-2 wherein said third part is made double stranded by hybridization with a complementary sequence to the signal tail.

5. A process to synthesize a probe according to claims 1-2, and 4 comprising:
a PCR step which is performed in the presence of a reverse primer which is similar to the first part of said probe according to claim 1, a forward primer which is similar to the fourth part of said probe according to claim 1, a mix of labeled and unlabeled dNTPs in order to generate labeling of the probe according to claim 1 and a template consisting of the following parts indicated in the 5' to 3' order: 1) a first part comprising a nucleotide sequence that is similar to a forward primer, 2) a second part comprising a nucleotide sequence of between 200 and 100,000 nucleotides composed of only 3 different types of nucleotides chosen from the 5 different types A, C, G, T or U, 3) optionally, a third part comprising the types of nucleotides which are not chosen in said second part, 4) a fourth part functioning as a spacer and comprising at least one nucleotide, and 5) a fifth part comprising a nucleotide sequence of between 14 and 200 nucleotides that is complementary to a reverse primer.

6. A process to synthesize a probe according to claim 3 comprising:
a PCR step according to claim 5 wherein said template comprises said first part and/or third part, and a second PCR step which is performed in the presence of a forward primer, a dNTPs mix which only contain nucleotides which are complementary to the 3 different types of nucleotides chosen in the second part of the template according to the first PCR step.

7. A process to synthesize a probe according to claim 4comprising: hybridization of the signal tail with a complementary sequence to the signal tail.

8. Use of a probe according to claims 1-4 to specifically detect small target sequences.

9. A kit comprising a probe as defined by claims 1-4.

10. An in vitro method to detect a methylation changes-induced single nucleotide polymorphism *in situ* and/or to distinguish methylation heterogeneity from hemi-methylation and mono-allelic methylation in a sample taken from a patient consisting of:
- providing a sample obtained from said patient,
- treating said sample with adequately dosed pepsin and/or protease K and/or HCL and/or detergent and/or ethanol to permeabilize samples and to remove proteins from said sample,
- incubating said sample with adequately dosed bisulfite reagents in the presence of a RNase inhibitor to create non-complementary single stranded DNA strands,
- incubating said samples with specifically designed blocking probes and/or DNA-protecting probes for at least one 1 hour, and
- incubating said sample with a specifically designed probe according to claims 1-4.

11. Use of a kit according to claim 9 to perform the method according to claim 10.

## Patentansprüche

1. Sonde, bestehend aus den folgenden von 5' nach 3' angeordneten Teilen: a) einem ersten Teil, der eine Nukleotidsequenz zwischen 14 und 200 Nukleotiden umfasst, die zu einer Zielsequenz komplementär und einem Rückwärtsprimer ähnlich ist, b) einem zweiten Teil, der als Spacer fungiert und wenigstens ein Nukleotid umfasst, das nicht zur Zielsequenz komplementär ist, c) einem dritten Teil, der eine Nukleotidsequenz zwischen 200 und 100 000 Nukleotiden umfasst, die nicht zur Zielsequenz komplementär ist, wobei sich die Nukleotide aus nur 3 unterschiedlichen Nukleotidtypen, die aus den 5 unterschiedlichen Typen A, C, G, T oder U gewählt sind, zusammensetzen und wobei 10 bis 100% der Nukleotide markiert sind, und d) einem vierten Teil, der eine Nukleotidsequenz umfasst, die nicht zur Zielsequenz, aber zu einem Vorwärtsprimer komplementär ist.

2. Sonde nach Anspruch 1, ferner umfassend einen fünften Teil, der die Nukleotidtypen umfasst, die im dritten Teil nicht gewählt sind.

3. Sonde nach einem der Ansprüche 1-2, wobei der dritte Teil durch PCR zum Doppelstrang gemacht wird.

4. Sonde nach einem der Ansprüche 1-2, wobei der dritte Teil durch Hybridisierung mit einer komplementären Sequenz zum Signalende zum Doppelstrang gemacht wird.

5. Verfahren zur Synthese einer Sonde nach Anspruch 1-2 und 4, umfassend:
einen PCR-Schritt, der in Gegenwart eines Rückwärtsprimers, der dem ersten Teil der Sonde nach Anspruch 1 ähnlich ist, eines Vorwärtsprimers, der dem vierten Teil der Sonde nach Anspruch 1 ähnlich ist, einer Mischung aus markierten und unmarkierten dNTP, um eine Markierung der Sonde nach Anspruch 1 zu erzeugen, und einer Matrize erfolgt, die aus den folgenden von 5' nach 3' angeordneten Teilen besteht: 1) einem ersten Teil, der eine Nukleotidsequenz umfasst, die einem Vorwärtsprimer ähnlich ist, 2) einem zweiten Teil, der eine Nukleotidsequenz zwischen 200 und 100 000 Nukleotiden umfasst, die sich aus nur 3 unterschiedlichen Nukleotidtypen, die aus den 5 unterschiedlichen Typen A, C, G, T oder U gewählt sind, zusammensetzen, 3) gegebenenfalls einem dritten Teil, der die Nukleotidtypen umfasst, die im zweiten Teil nicht gewählt sind, 4) einem vierten Teil, der als Spacer fungiert und wenigstens ein Nukleotid umfasst, und 5) einem fünften Teil, der eine Nukleotidsequenz zwischen 14 und 200 Nukleotiden umfasst, die zu einem Rückwärtsprimer komplementär ist.

6. Verfahren zur Synthese einer Sonde nach Anspruch 3, umfassend:
einen PCR-Schritt nach Anspruch 5, wobei die Matrize den ersten Teil und/oder dritten Teil umfasst, und einen zweiten PCR-Schritt, der in Gegenwart eines Vorwärtsprimers, einer dNTP-Mischung, die nur Nukleotide enthält, die zu den 3 unterschiedlichen Nukleotidtypen, die im zweiten Teil der Matrize gemäß dem ersten PCR-Schritt gewählt sind, komplementär sind.

7. Verfahren zur Synthese einer Sonde nach Anspruch 4, umfassend: Hybridisierung des Signalendes mit einer komplementären Sequenz zum Signalende.

8. Verwendung einer Sonde nach Anspruch 1-4 zum spezifischen Nachweisen kleiner Zielsequenzen.

9. Kit, umfassend eine Sonde gemäß Anspruch 1-4.

10. In-vitro-Methode zum Nachweisen eines durch Methylierungsänderungen induzierten Einzelnukleotid-Polymorphismus *in situ* und/oder zur Unterscheidung von Methylierungsheterogenität von Hemimethylierung und monoallelischer Methylierung in einer einem Patienten entnommenen Probe, umfassend:
- Bereitstellen einer vom Patienten erhaltenen Probe,
- Versetzen der Probe mit angemessen dosiertem bzw. dosierter Pepsin und/oder Protease K und/oder HCL und/oder Detergens und/oder Ethanol zum Permeabilisieren von Proben und Entfernen von Proteinen aus der Probe,
- Inkubieren der Probe mit angemessen dosieren Hydrogensulfitreagentien in Gegenwart eines RNase-Inhibitors zur Erzeugung nicht komplementärer einzelsträngiger DNA-Stränge,
- wenigstens 1 Stunde Inkubieren der Proben mit spezifisch konstruierten Blockiersonden und/oder DNA schützenden Sonden und
- Inkubieren der Probe mit einer spezifisch konstruierten Sonde nach Anspruch 1-4.

11. Verwendung eines Kits nach Anspruch 9 zur Durchführung der Methode nach Anspruch 10.

## Revendications

1. Sonde constituée par les parties suivantes, indiquées dans un ordre de 5' vers 3' : a) une première partie comprenant une séquence nucléotidique d'une longueur comprise entre 14 et 200 nucléotides qui est complémentaire à une séquence cible et qui est semblable à une amorce arrière, b) une deuxième partie fonctionnant comme espaceur et comprenant au moins un nucléotide qui n'est pas complémentaire à la séquence cible, c) une troisième partie comprenant une séquence nucléotidique d'une longueur comprise entre 200 et 100 000 nucléotides qui n'est pas complémentaire à ladite séquence cible, dans laquelle lesdits nucléotides sont composés de seulement 3 types différents de nucléotides, choisis parmi les 5 types différents A, C, G, T ou U, et dans laquelle de 10 à 100% desdits nucléotides sont marqués, et d) une quatrième partie comprenant une séquence nucléotidique qui n'est pas complémentaire à ladite séquence cible mais qui est complémentaire à une amorce avant.

2. Sonde, selon la revendication 1, comprenant en outre une cinquième partie comprenant les types de nucléotides qui ne sont pas choisis dans la troisième partie.

3. Sonde selon l'une quelconque des revendications 1 à 2, dans laquelle ladite troisième partie est rendue en double brin par une ACP.

4. Sonde selon l'une quelconque des revendications 1 à 2, dans laquelle ladite troisième partie est rendue en double brin par une hybridation avec une séquence complémentaire à la queue du signal.

5. Processus destiné à synthétiser une sonde selon les revendications 1 à 2 et 4 comprenant :
une étape d'ACP qui est exécutée en présence d'une amorce arrière qui est semblable à la première partie de ladite sonde selon la revendication 1, d'une amorce avant qui est semblable à la quatrième partie de ladite sonde selon la revendication 1, d'un mélange de dNTP marqués et non marqués dans le but de générer un marquage de la sonde, selon la revendication 1, ainsi que d'une matrice constituée par les parties suivantes, indiquées dans l'ordre de 5' vers 3' : 1) une première partie comprenant une séquence nucléotidique qui est semblable à une amorce avant, 2) une deuxième partie comprenant une séquence nucléotidique d'une longueur comprise entre 200 et 100 000 nucléotides composés de seulement 3 types différents de nucléotides, choisis parmi les 5 types différents A, C, G, T ou U, 3) en option, une troisième partie comprenant les types de nucléotides qui ne sont pas choisis dans ladite deuxième partie, 4) une quatrième partie fonctionnant comme espaceur et comprenant au moins un nucléotide, et 5) une cinquième partie comprenant une séquence nucléotidique d'une longueur comprise entre 14 et 200 nucléotides qui est complémentaire à une amorce arrière.

6. Processus destiné à synthétiser une sonde, selon la revendication 3, comprenant :
une étape d'ACP selon la revendication 5, dans laquelle ladite matrice comprend lesdites première partie et/ou troisième partie, ainsi qu'une deuxième étape d'ACP qui est exécutée en présence d'une amorce avant, d'un mélange de dNTP qui contient seulement des nucléotides qui sont complémentaires aux 3 types différents de nucléotides choisis dans la deuxième partie de la matrice selon la première étape d'ACP.

7. Processus destiné à synthétiser une sonde, selon la revendication 4, comprenant : une hybridation de la queue du signal avec une séquence complémentaire à la queue du signal.

8. Utilisation d'une sonde, selon les revendications 1 à 4, pour détecter spécifiquement de petites séquences cibles.

9. Trousse comprenant une sonde telle que définie par les revendications 1 à 4.

10. Procédé in vitro destiné à détecter un polymorphisme nucléotidique simple induit par des changements de la méthylation *in situ* et/ou destiné à distinguer une hétérogénéité de la méthylation à partir d'une hémi-méthylation et d'une méthylation mono-allélique dans un échantillon prélevé sur un patient, constitué par les étapes consistant à :
- fournir un échantillon obtenu à partir dudit patient,
- traiter ledit échantillon avec une pepsine et/ou une protéase K et/ou du HCl et/ou un détergent et/ou de l'éthanol, convenablement dosés, pour perméabiliser des échantillons et pour ôter des protéines dudit échantillon,
- incuber ledit échantillon avec des réactifs de bisulfite, convenablement dosés, en présence d'un inhibiteur de RNAse pour créer des brins d'ADN en simple brin non complémentaires,
- incuber lesdits échantillons avec des sondes bloquantes et/ou des sondes de protection de l'ADN, spécifiquement conçues, pendant au moins 1 heure et
- incuber ledit échantillon avec une sonde spécifiquement conçue selon les revendications 1 à 4.

11. Utilisation d'une trousse, selon la revendication 9, pour exécuter le procédé selon la revendication 10.


### SEQUENCE LISTING

<110> Universiteit Gent
<120> Probes and a methylation in situ hybridization assay
<130> P2015/050 EPNAT
<150> EP15176744.9
   <151> 2015-07-15
<160> 18
<170> PatentIn version 3.5
<210> 1
   <211> 24
   <212> DNA
   <213> Artificial sequence
<220>
   <223> Primer
<400> 1
   tttttagagg atttgaggga tagg 24
<210> 2
   <211> 24
   <212> DNA
   <213> Artificial sequence
<220>
   <223> Primer
<400> 2
   ctacctaatt ccaattcccc taca 24
<210> 3
   <211> 14
   <212> DNA
   <213> Homo Sapiens
<400> 3
   acmgtccatc mgct 14
<210> 4
   <211> 14
   <212> DNA
   <213> Homo Sapiens
<400> 4
   acmgtuuatc mgut 14
<210> 5
   <211> 12
   <212> DNA
   <213> Artificial sequence
<220>
   <223> Primer
<400> 5
   acgtccatcg ct 12
<210> 6
   <211> 12
   <212> DNA
   <213> Artificial sequence
<220>
   <223> Primer
<400> 6
   augtuuatug ut 12
<210> 7
   <211> 60
   <212> DNA
   <213> Homo Sapiens
<400> 7
   gaggcmgccm gccmgccmgc cmgctgccmg ccmgcacact gggatccmgc tcmggcagca 60
<210> 8
   <211> 42
   <212> DNA
   <213> Homo Sapiens
<400> 8
   cgaacgaatc ccaatatacg acgacaacga cgacgacgac gc 42
<210> 9
   <211> 42
   <212> DNA
   <213> Homo Sapiens
<400> 9
   cgccgccgcc gccgctaccg ccgcacacta aaatccgctc ga 42
<210> 10
   <211> 60
   <212> DNA
   <213> Homo Sapiens
<400> 10
   gaggcmgccm gccmgccmgc cmgctgccmg ccmgcacact gggatccmgc tcmggcagca 60
<210> 11
   <211> 42
   <212> DNA
   <213> Homo Sapiens
<400> 11
   caaacaaatc ccaatataca acaacaacaa caacaacaac ac 42
<210> 12
   <211> 42
   <212> DNA
   <213> Homo Sapiens
<400> 12
   caccaccacc accactacca ccacacacta aaatccactc aa 42
<210> 13
   <211> 126
   <212> DNA
   <213> Homo Sapiens
<400> 13
   tggttaaggt tattggggtg tttttggaga tttaggggtt aattggttgg tgtttatatt 60
   tatttgtggg gattagtgtt gtggtggaga agagtaatag tagaagttgg agttggagtt 120
   tgggag 126
<210> 14
   <211> 115
   <212> DNA
   <213> Homo Sapiens
<400> 14
   cractccrac ttctactatt actcttctcc rccrcracac taatccccac aaataaatat 60
   aaacaccrac crattaaccc ctaaatctcc raaaacaccc caataacctt aacca 115
<210> 15
   <211> 32
   <212> DNA
   <213> Artificial sequence
<220>
   <223> Primer
<400> 15
   gttgtgtaat tcgttggatg cggattaggg cg 32
<210> 16
   <211> 32
   <212> DNA
   <213> Artificial sequence
<220>
   <223> Primer
<400> 16
   cgccctaatc cgcatccaac gaattacaca ac 32
<210> 17
   <211> 12
   <212> DNA
   <213> Artificial sequence
<220>
   <223> Example of part of template for signal tail
<400> 17
   acgacggcca aa 12
<210> 18
   <211> 12
   <212> DNA
   <213> Artificial sequence
<220>
   <223> Example of part of signal tail
<400> 18
   tgctgccggt tt 12